# EUROPEAN PATENT SPECIFICATION

(11) **EP 1 520 525 B1**
(45) Date of publication and mention of the grant of the patent: **24.03.2010**
(21) Application number: 04256039.1
(22) Date of filing: 30.09.2004
(51) Int. Cl.: A61B 17/072

(54) **Apparatus for attaching a surgical buttress to a stapling apparatus**
Gerät zum Befestigen einer chirurgischen Versteifung an einem Klammergerät
Dispositif pour attacher une pièce d'appui à une agrafeuse chirurgicale

(30) Priority: 30.09.2003 US 674303
(43) Date of publication of application: 06.04.2005
(73) Proprietor: ETHICON, INC., Somerville, NJ 08876 (US)
(72) Inventor: Dalessandro, David A., Scotch Planes, New Jersey 07076 (US); Li, Zhigang, Hillsborough, New Jersey 08844 (US); Scopelianos, Angelo G., Whitehouse Station, New Jersey 08889 (US)
(74) Representative: Mercer, Christopher Paul

(56) References cited:
- EP-A- 1 064 883
- US-A- 5 752 965
- US-A- 5 902 312

## Description

### FIELD OF THE INVENTION

This invention relates to an apparatus for releasably attaching a surgical buttress, or pledget, to the working surfaces of a surgical stapling device.

### BACKGROUND OF THE INVENTION

Surgical stapling is often employed by surgeons to apply a plurality of laterally spaced rows of staples on opposite sides of a tissue cut for the purpose of approximating body organs and tissues such as lung, esophagus, stomach, duodenum and other body organs. The surgical stapling devices generally consist of a pair of cooperating elongated jaw members. One of the jaw members includes a staple cartridge with at least two laterally spaced rows of staples and the other jaw member includes an anvil with staple closing depressions in alignment with the rows of staples in the cartridge. A pusher block is directed longitudinally along the jaws to sequentially eject staples from the cartridges in a manner that closes the staples against the anvil to form laterally spaced lines of staples through tissue that is gripped between the jaws. A knife is associated with the pusher block so as to move forward along the jaws to cut the tissue along the line between the previously formed staple rows. A common drawback of the use of known surgical stapling devices is that a certain amount of tissue tearing, or fluid and air leakage, may be experienced along the surgical stapling line.

U.S. Pat. No. 5,964,774 discloses methods and apparatus for achieving hemostasis and pneumostasis along a staple line by utilizing a pledget material or bolstering material positioned adjacent to at least one surface of the tissue.

An attempt to produce a reinforced surgical stapling line is taught in U.S. Pat. No. 5,263,629. Here, a disposable anvil and fastener cartridges are provided having pledget material disposed thereon for producing a reinforced surgical stapling line. However, a substantial drawback exists with using disposable cartridges and anvils, such as limited ability to retrofit other types of surgical stapling devices (e.g. those having permanent or non-disposable anvil and cartridge portions).

Another attempt at producing a reinforced surgical stapling line is taught in U.S. Pat. No. 5,441,193. A sheet of material having a concave shape along the longitudinal axis is attached to one or both of the anvil and cartridge portions for biasing the subject body tissue together when the anvil and cartridge portions of the surgical stapling device are closed onto the body tissue.

In U.S. Patent No. 5,397,324, Carroll et al. disclose a surgical stapling device having a pair of flexible body-absorbable or non-absorbable pads which are captively held on the staple cartridge during the positioning of the subject body tissue between the anvil and staple cartridge and releasable such that the surgical staples upon ejection from the cartridge will penetrate through the pads, the tissue, and bend against the anvil.

Yet another effort to produce reinforced surgical stapling lines is disclosed in U.S. Pat. No. 5,503,638. Here it is taught to provide a U-shape buttress member having pledget material removably affixed between the parallel sides of the buttress member via filament stitching.

U.S. Pat. No. 5,814,057 describes a supporting element having a cylinder or prism shape for staple region comprising a fabric-like object made of a biodegradable material integrated with stretchable textile at both ends. The two materials are joined by sewing yarn whose ends are extended outside and placed at an integration section in plain stitch.

U.S. Pat. No. 5,702,409 describes a device with at least one face proportioned to reinforce surgical staples and walls extending from either side of the operative face comprised of the same material. The face and the walls form a tube having a generally rectangular cross sectional shape.

A retainer assembly having an alignment frame and a separate pressure equalization member is disclosed in U.S. Pat. No. 5,752,965. This combination of the pressure equalization member with the alignment frame is necessary because of the lack of deformability and resiliency found in the pledget itself, which is comprised of tanned bovine pericardium.

While various methods and apparatus for providing reinforced surgical staple lines as described in the prior art exist, still, a need exists for a device and method for attaching a surgical buttress to a stapling device to produce reinforced stapling lines, which has universal applicability with any type and size of surgical stapling device and which facilitates and simplifies such methods.

### SUMMARY OF THE INVENTION

The present invention is directed to an apparatus as defined in claim 1 for equipping a surgical stapling device with a surgical buttress in order to provide reinforced surgical fastener suture lines. The apparatus comprises an alignment means, an elastomeric foam buttress and a means for retaining the foam buttress within the alignment. The alignment means comprises a substantially planar frame having a first surface and a second surface apposed to the first surface, first and second guide channel walls extending upwardly and substantially perpendicular from the first and second surfaces of the planar frame, respectively, and means for receiving the surgical buttress comprising a first surface and a second surface apposed to the first surface. The guide channel walls in cooperation with the receiving means provide guide channels for receipt of elements of the stapling device. The alignment means is formed as a unitary body comprising the frame, guide channel walls and receiving means.

The elastomeric foam surgical buttress comprises an elastomeric polymer foam, has a first surface for contacting the receiving means and a second surface apposed to the first surface for contacting the stapling device, and is disposed on the receiving means such that upon contact with the stapling device, the buttress may be released from the receiving means. The foam buttress is releasably retained on the receiving means either by adhesive or other mechanical means, such as retention channels integral with the alignment means. Prior to placement of the apparatus in contact with the stapling device, an adhesive preferably is disposed on the second surface of the buttress so that upon contact with the stapling device, the buttress is transferred to the stapling device in a releasable relationship.

### BRIEF DESCRIPTION OF THE FIGURES

Figure 1 is a perspective view of an alignment means utilized in an apparatus of the present invention;
Figure 2a is a top plan view of a surgical buttress utilized in an apparatus of the present invention;
Figure 2b is a top plan view of a surgical buttress utilized in an apparatus of the present invention;
Figure 3 is a perspective view of an apparatus of the present invention depicting an alignment means in cooperation with a surgical buttress for producing reinforced surgical stapling lines;
Figure 4 is a perspective view illustrating the method of engagement between a surgical stapling device and an apparatus of the present invention; and
Figs. 5a-5c are cross-sectional views taken along section line A-A of Figure 4.

### DETAILED DESCRIPTION OF THE INVENTION

The apparatus of the present invention is capable of loading a surgical stapling device with a surgical buttress article so that the stapling devices will produce reinforced surgical stapling lines when staples are delivered to the body from the stapling device. First and second articles of buttress material as disclosed herein below are disposed on apposed surfaces of a receiving means and within the guide channel of the alignment means of the present invention such that the apposed jaw members of the stapling device automatically will be aligned with the foam buttress articles when the alignment means is placed in cooperation with the stapling device.

The buttresses may be retained within the alignment means by a biocompatible adhesive placed between the receiving means and the buttress. Alternately, the alignment means itself may be constructed such that the means for retaining the buttress within the alignment means is an integral feature of the alignment means. For example, the alignment means may be machined or otherwise formed or molded to include channels, or grooves, or functionally equivalent structures, as shown herein. In such a case, the buttress is configured to fit into or otherwise cooperate with the retaining means to facilitate retention of the buttress within the alignment means.

An adhesive is applied to an exposed surface of the surgical buttress so as to be contactable by the jaw members upon engagement of the alignment means by the stapling device. The alignment means containing the buttress articles is engaged by the jaw members of the surgical stapling device and the jaws are closed so that the apposed jaw members contact the adhesive disposed on the buttress articles. The jaws are opened and the alignment means is removed from the stapling device. The foam surgical buttress is retained on the jaw member in a releasably-attached relationship, such that upon firing of the stapling device, the surgical buttress is released from the jaw member and disposed along the stapling line, thereby providing reinforced surgical stapling lines.

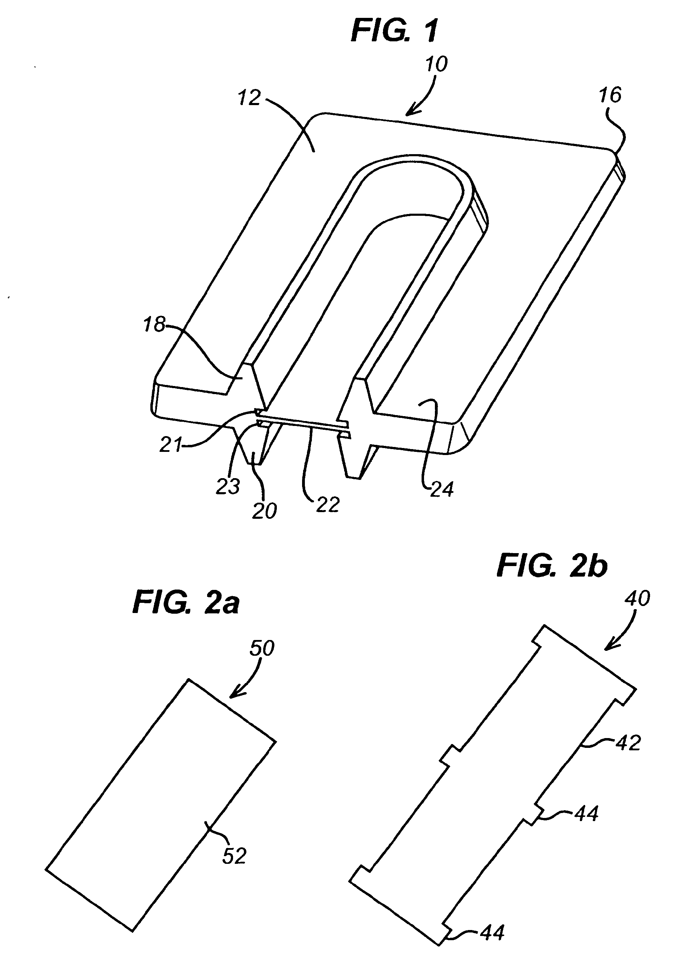

In a preferred embodiment of the invention, Figure 1 shows alignment means 10, which includes substantially planar frame 16 having apposed first surface 12 and second surface 14 (not shown), first guide channel wall 18, second guide channel wall 20, and means for receiving 22 a surgical buttress article within alignment means 10. Alignment means 10 is a single, unitary structure comprising first 18 and second 20 guide channel walls extending substantially perpendicular from apposed surfaces 12 and 14 of planar frame 16, respectively and receiving means 22, comprising first surface 24 and apposed second surface 26 (not shown). Receiving means 22 is substantially horizontal and continuous with planar frame 16. First 18 and second 20 guide channels, respectfully, define the perimeter of receiving means 22. Guide channel walls 18 and 20 and receiving means 22 together define guide channels to ensure proper alignment of apposed jaws of a stapling device when placed engaged by alignment means 10. Though shown as approximately equivalent in dimensions in Figure 1, first 18 and second 20 guide channel walls are dimensioned to regulate the engagement of the apposed working surfaces of a surgical stapling device.

Preferably, alignment means 10 further comprises means for retaining the foam surgical buttress in alignment means 10 and in contact with receiving means 22. As shown, first 21 and second 23 retention channels, or grooves, are formed integral with and between first 18 and second 20 guide channel walls, respectively, and receiving means 22.

As shown in Figure 2a, surgical buttresses 50 utilized in the present invention, having first 52 and second 54 (not shown) apposed surfaces, may be rectangular in shape and are dimensioned to fit the apposed working surfaces of a surgical stapling device. Buttress 50 may be constructed such that the width is sufficient to fit within and cooperate with retention channels 21, 23 to retain buttress 50 within alignment means 10 and on receiving mean 22.

In a preferred, embodiment shown in Figure 2b, surgical buttress 40 comprises rectangular body 42, with a series of tabs 44 extending laterally from body 42 in appropriate number and at appropriate position to fit within and cooperate with retention channels 21, 23. Thus, the cooperation of tabs 44 and retention channels 21, 23 provide retention of surgical buttress 40 within alignment means 10, while also allowing surgical buttress 40 to be non-obstructively removed from alignment means 10. Preferably, three tabs 44 are disposed on each side of surgical buttress 40. Though shown on Figure 2B as square, tabs 44 could be, rectangular, half circular, trapezoid or triangular.

Surgical buttresses utilized in the present invention comprise a compliant, compressible, biodegradable material capable of uniformly distributing pressure along the staple line to cause substantial hemostasis or pneumostasis along the tissue cut. The material may be in the form of a foam, a porous membrane, a non-woven, expanded polytetraethylene, and the like. For soft tissue applications a compliant, compressible, biodegradable foam material is preferred. The buttress material also provides a medium for the staples to hold onto in the case of thin or diseased tissue. The material also absorbs impact and reduces trauma. As will be discussed later, the compliant, compressible nature of the biodegradable foam material is particularly important to the present invention in that it provides for a more even distribution of pressure between the working surfaces of a surgical stapling device and the means for receiving the surgical buttress within the alignment means when the surgical buttress is being disposed on the working surfaces of the surgical stapling device.

Suitable materials used to make foam buttresses for use in the present invention include biocompatible elastomeric polymers. Preferably, the polymer also will be biodegradable. Examples of suitable biodegradable elastomers are described in U. S. Pat. No. 5,468,253, . Preferably, the biodegradable, biocompatible elastomers are based on aliphatic polyesters, including but not limited to those selected from the group consisting of elastomeric copolymers of epsilon-caprolactone and glycolide (preferably having a mole ratio of epsilon-caprolactone to glycolide of from about 35/65 to about 65/35, more preferably 35/65 to 45/55); elastomeric copolymers of epsilon-caprolactone and lactide (including L-lactide, D-lactide, blends thereof or lactic acid copolymers (preferably having a mole ratio of epsilon-caprolactone to lactide of from about 35/65 to about 65/35 and more preferably 35/65 to 45/55, or from about 85/15 to about 95/5); elastomeric copolymers of para-dioxanone (1,4- dioxan-2-one) and lactide (including L-lactide, D-lactide and lactic acid (preferably having a mole ratio of para-dioxanone to lactide of from about 40/60 to about 60/40); elastomeric copolymers of epsilon-caprolactone and para-dioxanone (preferably having a mole ratio of epsilon-caprolactone to para-dioxanone of from about 30/70 to about 70/30); elastomeric copolymers of para-dioxanone and trimethylene carbonate (preferably having a mole ratio of para-dioxanone to trimethylene carbonate of from about 30/70 to about 70/30); elastomeric copolymers of trimethylene carbonate and glycolide (preferably having a mole ratio of trimethylene carbonate to glycolide of from about 30/70 to about 70/30); elastomeric copolymer of trimethylene carbonate and lactide (including L-lactide and D-lactide) and lactic acid copolymers (preferably having a mole ratio of trimethylene carbonate to lactide of from about 30/70 to about 70/30).

The elastomeric polymers and copolymers will have an inherent viscosity of from about 1.2 dL/g to about 4 dL/g, preferably an inherent viscosity of from about 1.2 dL/g to about 2 dL/g and most preferably an inherent viscosity of from about 1.4 dL/g to about 2 dL/g as determined at 25° C. in a 0.1 gram per deciliter (g/L) solution of polymer in hexafluoroisopropanol (HFIP).

Preferably, the elastomeric polymers will exhibit a high percent elongation and a low modulus, while possessing good tensile strength and good recovery characteristics. In the preferred embodiments of this invention, the elastomeric polymers from which the surgical buttresses are formed will exhibit a percent elongation greater than about 200, preferably greater than about 500. It will also exhibit a modulus (Young's Modulus) of less than about 27.6 MN/m₂ (4000 psi), preferably less than about 138 MN/m₂ (20,000 psi). Their properties, which measure the degree of elasticity of the biodegradable elastomeric polymer, are achieved while maintaining a tensile strength greater than about 3,45 MN/m₂ (500 psi), preferably greater than about 6,9 MN/m₂ (1,000 psi); and a tear strength of greater than about 50 lbs/inch, preferably greater than about 0,55 MN/m₂ (80 lbs/inch).

Foam materials comprising the elastomeric polymers may be formed by lyophilization, supercritical solvent foaming (i.e., as described in EP 464,163B1), gas injection extrusion, gas injection molding, or casting with an extractable material (i.e., salts, sugar or any other means known to those skilled in the art). Currently it is preferred to prepare biodegradable, biocompatible elastomeric foams by lyophilization. One suitable method for lyophilizing elastomeric polymers to form foam buttresses according to the present invention is described in U.S. Pat. No. 6,355,699, . Pharmaceutically active compounds may be incorporated into the foam buttress to further treat the patient, including but not limited to antibiotics, antifungal agents, hemostatic agents, antiinflammatory agents, growth factors and the like.

The aliphatic polyesters generally are prepared by a ring-opening polymerization of the desired proportions of one or more lactone monomers in the presence of an organometallic catalyst and an initiator at elevated temperatures. The organometallic catalyst preferably is a tin-based catalyst, e.g. stannous octoate, and is present in the monomer mixture at a molar ratio of monomer to catalyst ranging from about 15, 000/1 to about 80,000/1. The initiator typically is an alkanol (such as 1-dodecanol), a polyol (such as 1,2-propanediol, 1,3-propanediol, diethylene glycol, or glycerol, poly(ethylene glycol)s, poly(propylene glycol)s and poly(ethylene-co-propylene glycol)s), a hydroxyacid, or an amine, and is present in the monomer mixture at a molar ratio of monomer to initiator ranging from about 100/1 to about 5000/1. The polymerization typically is carried out at a temperature range from about 80°C to about 220°C, preferably 160°C to 190°C, until the desired molecular weight and viscosity are achieved.

Suitable bioabsorbable releasable adhesives that may be used according to the present invention include cellulosic and aliphatic ester homopolymers and copolymers made from polymers of the formula:

[-O-R¹¹-C(O)-]_{Y},

wherein R¹¹ is selected from the group consisting of -CR¹²H-, -(CH₂)₃-0-, -CH₂-CH₂-O-CH₂-, CR¹²H-CH₂, -(CH₂)₄-, -(CH₂)_{z}-O- and -(CH₂)_{z},-C(O)-CH₂-; R¹² is hydrogen or methyl; z is an integer in the range of from 1 to 7; and y is an integer in the range of from about 10 to about 20,000; blends of a viscous PEG liquid and a low melting solid PEG (solid at room temperature that melts at less than about 60°C); biocompatible monosaccharides, disaccharides and polysaccharides (such as pectin) that may be mixed with a plasticizer (such as glycerine) to form a tacky adhesive and biocompatible proteins (such as gelatin) that may mixed with a plasticizer (such as glycerine) to form a tacky adhesive.

Many nontoxic bioabsorbable aliphatic ester polymers that are semi-crystalline solids or tacky liquids at room temperature may be used as a releasable adhesive. The releasable adhesive of this invention are generally characterized as being flowable at body temperature (37°C) and preferably will flow at room temperatures (25°C). Most preferably these liquids will have a low yield point to avoid migration of the polymer. Examples of suitable tacky liquid copolymers are contained in U.S. Patent Application Serial No. 08/746,180, filed November 6, 1996. Additionally, tacky microdispersions may also be used such as those described in U.S. Patent No. 5,599,852, .

In particular liquid copolymers composed of in the range of from about 65 mole percent to about 35 mole percent of epsilon-caprolactone, trimethylene carbonate, ether lactone (which for the purpose of this invention is defined to be 1,4-dioxepan-2-one and 1,5-dioxepan-2-one) repeating units or combinations thereof with the remainder of the polymer being a plurality of second lactone repeating units are preferred. The second lactone repeating units will be selected from the group consisting of glycolic acid repeating units, lactic acid repeating units, 1,4-dioxanone repeating units, 6,6-dialkyl-1,4-dioxepan-2-one, combinations thereof and blends thereof. Additionally, epsilon-caprolactone, trimethylene carbonate, or an ether lactone may be copolymerized to provide a liquid copolymer. Preferred polymers for use as particulate solids are bioabsorbable polymers including homopolymers of poly(epsilon-caprolactone), poly(p-dioxanone), or poly(trimethylene carbonate), copolymers of epsilon-caprolactone and trimethylene carbonate, copolymers of epsilon -caprolactone and a plurality of second lactone repeating units. The second lactone repeating units may be selected from the group consisting of glycolic acid repeating units, lactic acid repeating units, 1,4-dioxanone repeating units, 1,4-dioxepan-2-one repeating units, 1,5-dioxepan-2-one repeating units and combinations thereof. The copolymers of epsilon - caprolactone will preferably be composed of from 99 mole percent to 70 mole percent epsilon-caprolactone with the remainder of the polymer being a plurality of second lactone repeating units.

The polymers may be linear, branched, or star branched; block copolymers or terpolymers; segmented block copolymers or terpolymers. These polymers will also be purified to substantially remove unreacted monomers that may cause an inflammatory reaction in tissue.

Preferred liquid copolymers for use as the releasable adhesive are composed of in the range of from about 65 mole percent to about 35 mole percent epsilon-caprolactone or an ether lactone repeating unit with the remainder of the copolymer being trimethylene carbonate repeating units. Examples of suitable terpolymers are terpolymers selected from the group consisting of poly(glycolide-co-epsilon-caprolactone-co-p-dioxanone) and poly(lactide-co-epsilon-caprolactone-co-p-dioxanone) wherein the mole percent of epsilon-caprolactone repeating units is from about 35 to about 65 mole percent.

Preferred are terpolymers having in the range of from 40 to 60 mole percent of epsilon-caprolactone repeating units. Examples of liquid copolymer for use as the releasable adhesive may be selected from the group consisting of poly(epsilon-caprolactone-co-trimethylene carbonate), poly(lactide-co-trimethylene carbonate), poly(epsilon-caprolactone-co-p-dioxanone), poly(trimethylene carbonate-co-p-dioxanone), poly(epsilon-caprolactone-co-lactide), poly(lactide-co-1,5-dioxepan-2-one), and poly(1,5-dioxepan-2-one-co-p-dioxanone), poly(lactide-co-1,4-dioxepan-2-one), and poly(1,4-dioxepan-2-one-co-p-dioxanone). The mole percent of epsilon-caprolactone, trimethylene carbonate or ether lactone repeating units in these polymers should be in the range of from about 35 to about 65 mole percent and preferably in the range of from 40 to 60 mole percent. Most preferably these liquid polymers will be statistically random copolymers. These polymers will also be purified to substantially remove unreacted monomers that may cause an inflammatory reaction in tissue.

The polymers used as the releasable adhesive should have an inherent viscosity as determined in a 0.1 g/dL solution of hexafluoroisopropanol (HFIP) at 25°C ranging from about 0.1 dL/g to about 0.8 dL/g, preferably from about 0.1 dL/g to about 0.6 dL/g, and most preferably from 0.15 dL/g to 0.25 dL/g for liquid polymers.
Additionally, blends of liquid and solid polyethylene glycols (PEG) may be used as releasable adhesives. The liquid PEG may have a molecular weight from about 200 to about 600. The solid PEG may have a molecular weight from about 3400 to about 10,000. Generally it is theorized, but in no way limits the scope of this invention, that the low molecular weight liquid PEG plasticizes the solid PEG to render the solid PEG tacky. Consequently the majority of the composition should be the solid PEG and preferably between about 50 and about 80 percent by weight of the composition will be solid PEG. For example, a liquid polyethylene glycol with molecular weight of 400 (PEG 400) may be blended with a solid polyethylene glycol with a molecular weight of about 2,000 (PEG 2000). The ratio of PEG 400 to PEG 2000 may vary from about 40:60 to about 30:70. These blends may be formed by mixing the liquid PEG and the solid PEG with constant stirring in a heated water bath until the solid melts and a clear liquid solution is formed. After these solutions are allowed to cool and the resulting mixture may be tested for tackiness and used if the desired tackiness is obtained used in the present invention.

Alternatively biocomptaible monosaccharides, disaccharides, polysaccharides or proteins can be used with a biocompatible plasticizer such as glycerine to form tacky films in the presence of water. These materials may be applied to the surface of the buttress material and activated by applying water before contacting with the staple applier.

The amount of releasable adhesive that will be applied depends on a variety of factors such as the releasable adhesive used the desired degree of resistance desired for the foam to release and the geometry of its application. Those skilled in the art will readily be able to determine the appropriate amount of releasable adhesive to apply to achieve the desired release profile.

Alignment means utilized in the present invention may be fabricated from metal or plastic. The alignment means may be machined, molded or otherwise formed by methods readily know to those skilled in the art of making such parts or devices. Preferably the alignment means is made from a thermoset or thermoplastic polymer and is made by injection or compression molding. Any biocompatible polymer that is machinable, moldable or otherwise formable may be employed. Examples include, without limitation, polyamides, polyethylene, polypropylene, polytetraflouroethylene, polycarbonate or polyoxymethylene.

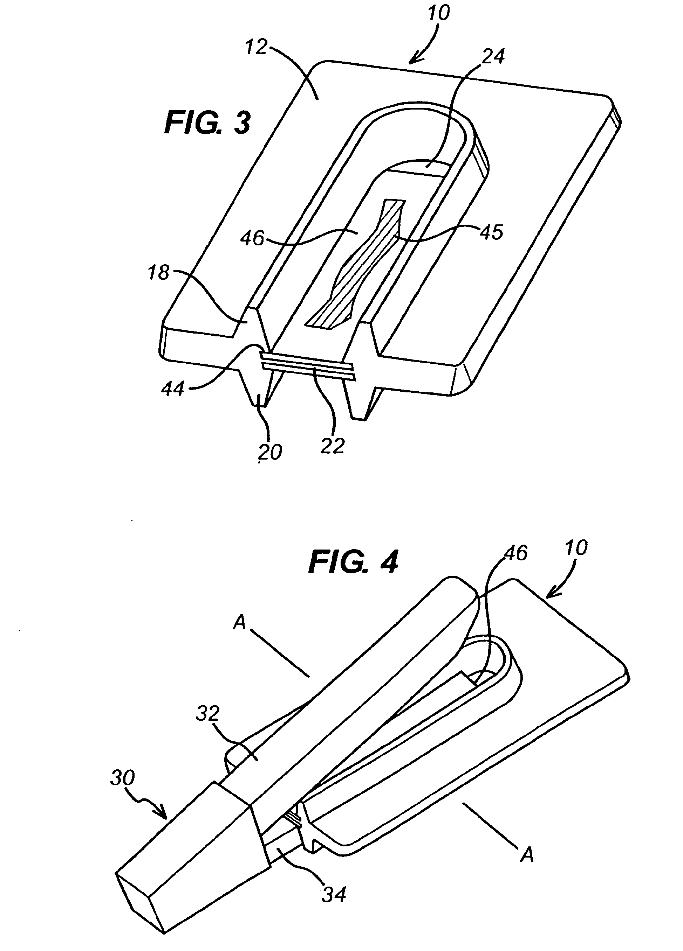

Figure 3 shows alignment means 10 in combination with two articles of surgical buttress as shown in Figure 2B. First surgical buttress 46 is disposed on first surface 24 of receiving means 22, with tabs 44 disposed in first retention channel 21. Second surgical buttress piece 48 is disposed on second surface 26 (not shown) of receiving means 22, with tabs 44 disposed in second retention channel 23. Surgical buttress pieces 46, 48 are designed to be positioned on opposed sides of receiving means 22 by sliding tabs 44 of surgical buttress 46, 48 into retention channels 21, 23, respectively, so as to be generally in line with first 18 and second 20 guide channel walls, respectively. Arranged as such, first 46 and second 48 surgical buttresses will be lined up with the apposed jaw members of a surgical stapling device when the surgical stapling device is registered within the guide channels formed by first 18 and second 20 guide channel walls and receiving means 22, respectively.

Adhesive 45 is disposed on the exposed surfaces of first surgical buttress 46 and second surgical buttress 48 (not shown). Adhesive 45 is disposed on the exposed surfaces of both surgical buttress 46 and 48 in an amount effective to provided a releasable attachment between surgical buttresses 46,48 and apposed working surfaces of a surgical stapling device to allow buttresses pieces 46,48 to be temporarily positioned on the working surfaces of the device prior to use of the device in surgical procedures for approximating body organs and tissues such as lung, esophagus, stomach, duodenum and other body organs. In a preferred embodiment of the present invention, adhesive 45 is a tacky liquid or gel, which is biocompatible with bodily tissue and preferably degradable.

First 46 and second 48 surgical buttresses are positioned on alignment means 10 such that first 18 and second 20 guide channel walls will direct the apposed working surfaces of a surgical stapling device into contact with surgical buttresses 46,48 when the surgical stapling device is clamped down onto alignment means 10. This effectively ensures for a consistent method of engagement between a particular surgical stapling device and alignment means 10 such that surgical buttresses 46,48 always will be applied to the apposed working surfaces of the surgical stapling device in the desired manner. This also allows a physician to quickly and easily retrofit an existing surgical stapling device to produce reinforced surgical stapling lines without undertaking painstaking efforts to properly position the buttress material on the surgical stapling device, thereby minimizing the costs associated with producing fortified surgical stapling lines.

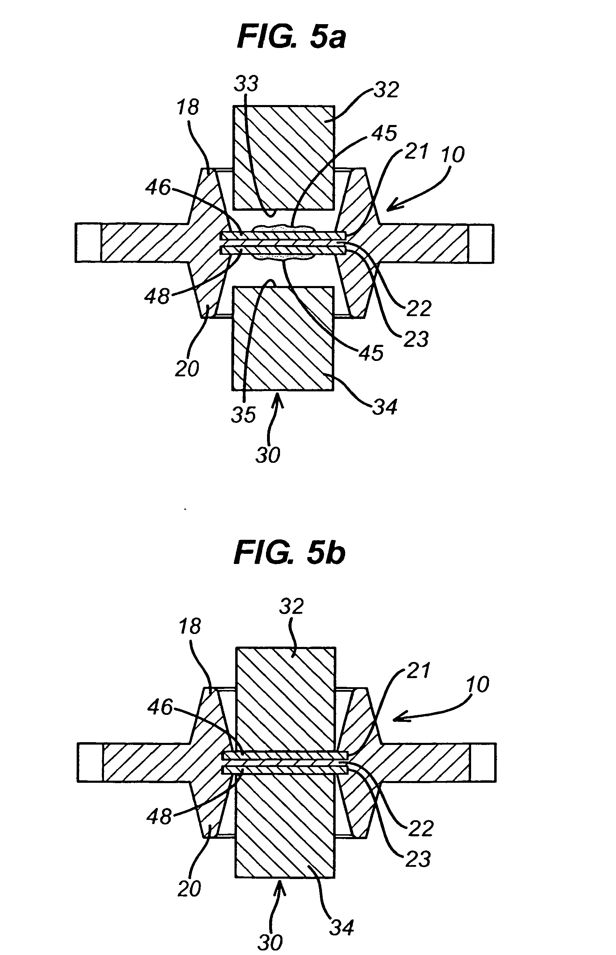

Figures 4 and 5a-c display the method of engagement between a typical surgical stapling device 30 and alignment means in combination with surgical buttresses 46,48. Surgical stapling device 30 comprises first 32 and second 34 jaw members. First 32 and second 34 jaw members have first and second apposed working surfaces 33 and 35, respectively. It is important to note that the embodiment of surgical stapling device 30 depicted is not limiting to the scope of the present invention, as there are a number of surgical stapling devices known in the art that will work with the invention disclosed herein.

The steps for engagement of alignment means 10 and stapling device 30 are summarized as follows. First 33 and second 34 jaw members are engaged within first 18 and second 20 guide channel walls, respectively. First 32 and second 34 jaw members are clamped together so as to compress first 46 and second 48 surgical buttresses into contact with receiving means. Jaw members 32,34 are opened from their previously closed position and remove first 46 and second 48 surgical buttresses from alignment means 10, such that first 46 and second 48 surgical buttresses are temporarily and releasably attached to apposed working surfaces 33,35 of first 32 and second 34 jaw members, respectively.

Details of the method are shown in Figures 5a to 5c. Figure 5a is a cross-section of Figure 4 taken along line A---A. The figure shows first surgical buttress piece 46 disposed on first surface 24 of receiving means, and second surgical buttress 48 disposed on second surface 26 of receiving means 22. Adhesive 45 is disposed on the exposed surfaces of both surgical buttresses 46,48 in an amount effective to provided a releasable attachment between surgical buttresses 46,48 and apposed working surfaces 33,35. A releasable attachment will allow buttresses 46,48 to be transferred from alignment means 10 and temporarily positioned on apposed working surfaces 33,35 of jaw members 32,34, respectively, prior to use of the device in surgical procedures for approximating body organs and tissues such as lung, esophagus, stomach, duodenum and other body organs.

Figure 5A also shows first 32 and second 34 jaw members of surgical stapling device 30 positioned within the guide channels formed by first 18 and second 20 guide channel walls, respectively.

After positioning jaw members 32,34 within the channels, the next step is to clamp the jaws down onto surgical buttresses 46,48. Figure 5b shows first 32 and second 34 jaw members clamped down into contact with adhesive-laden surgical buttresses 46,48. The surgical stapling device is maintained in this closed position for a period of time sufficient to adhere surgical buttresses 46,48 to apposed working surfaces 33,35. The deformable and resilient nature of surgical buttresses 46,48 is particularly important to the present invention in that it provides for an even distribution of pressure between apposed working surfaces 33,35 of surgical stapling device 30 and surgical buttresses 46,48 when first 32 and second 34 jaw members are clamped down into contact with adhesive-laden surgical buttress pieces 46,48. The uniform distribution of pressure ensures that surgical buttresses 46,48 will more readily conform to the shape and contour of apposed working surfaces 33,35 during the step of closing the surgical stapling device 30 about alignment means 10.

At this point, surgical buttresses 46,48 cannot be removed from alignment means 10 by sliding them along the length of receiving means 22 because the sliding could crimp and even tear surgical buttresses 46,48.

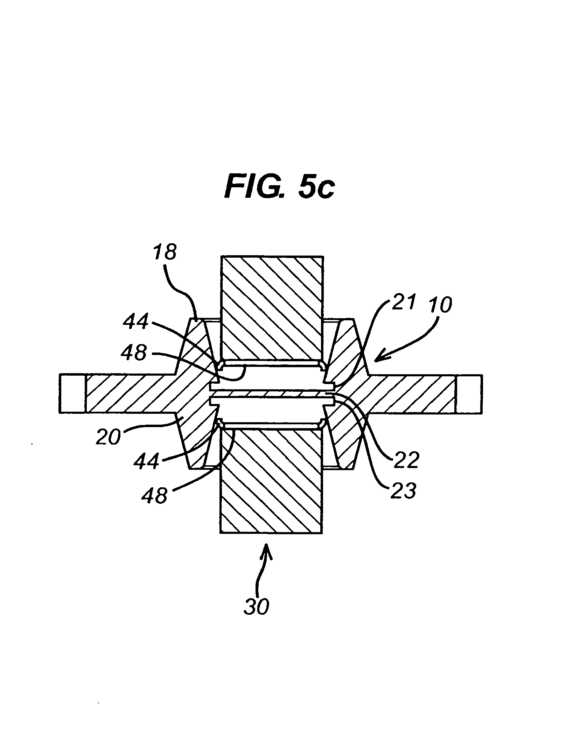

The final step in the method of engagement between surgical stapling device 30 and alignment means 10 in combination with surgical buttresses pieces 46,48 is to open jaw members 32,34 from their clamped position. Figure 5c shows jaw members 32,34 in their reopened position. The figure shows that when surgical stapling device 30 is opened from the previously clamped position, surgical buttresses 46,48 are attached to the jaw members 32,34, and can be removed from alignment means 10.

Tabs 44 of surgical buttresses 46,48 are bent as shown in Figure 5C and drawn out of retention grooves 21,23, resulting in no resistance to movement of surgical buttresses 46,48.

Surgical stapling device 30 is now equipped with surgical buttresses 46,48 such that apposed working surfaces 33,35 may be positioned about a section of body tissue to form a reinforced surgical stapling line in accordance with the present invention.

The present invention may be advantageously provided within a vacuum thermoformed plastic container that is sterilized and hermetically sealed so as to provide alignment means 10, adhesive 45, and surgical buttresses 46,48 in a convenient and ready-to-use condition. In a preferred embodiment, first 46 and second 48 surgical buttresses are loaded into alignment means 10 at the manufacturing site. In this arrangement, the present invention can be quickly and efficiently employed to prepare a surgical stapling device for producing reinforced surgical stapling lines by carrying out the following steps: (1) Removing the buttress carrier from the sterile package; (2) Removing the adhesive tube from the sterile package; (3) Spreading the adhesive onto the first and second surgical buttresses as they are positioned on the alignment means; (4) Positioning the apposed working surfaces of a surgical stapling device in general alignment with the first and second guide channel walls of the alignment means; (5) Closing the jaws of the surgical stapling device onto the adhesive-laden first and second surgical buttresses; (6) Open the surgical stapling device from the previously closed position so as to remove the first and second surgical buttresses from the alignment means; (8) Positioning the apposed working surfaces of the surgical stapling device over a designated portion of body tissue; (9) Closing the jaw members of the surgical stapling device into a compressed relation about the subject body tissue; (10) Firing the surgical stapling device to form a reinforced surgical stapling line; (11) Opening the jaw members of the surgical stapling device; and (12) Removing the surgical stapling device from the site of the surgical stapling line.

## Claims

1. An apparatus for equipping a surgical stapling device (30) to provide reinforced surgical fastener suture lines, comprising:
an alignment means (10) that comprises a substantially planar frame (16), said frame comprising:
a first surface (12) comprising a first guide channel wall (18) extending therefrom, and a second surface (14) apposed to said first surface and comprising a second guide channel wall (20) extending therefrom; and
a receiving means (22) comprising first (24) and second (26) apposed surfaces,
said first guide channel wall (18) and said first surface (24) of said receiving means (22) defining a first guide channel and said second guide channel wall (20) and said second surface (26) of said receiving means (22) defining a second guide channel,
a first surgical buttress (46, 40, 50) comprising a first (52) surface for contacting said receiving means (22) and a second (54) surface apposed to said first (52) surface for contacting said stapling device (30), said first buttress (46, 40, 50) disposed within said first guide channel and on said first surface (24) of said receiving means (22),
a second surgical buttress (48, 50) comprising a first (52) surface for contacting said receiving means (22) and a second (54) surface apposed to said first (52) surface for contacting said stapling device (30), said second buttress (48, 40, 50) disposed within said second guide channel and on said second surface (26) of said receiving means (22); and
means for retaining said first (46, 40, 50) and second (48, 40, 50) buttresses on said first (24) and second (26) surfaces of said receiving means (22), **characterized in that** the alignment means (10) is formed as a unitary body and the first (46,40,50) and second surgical buttress (48) are made of an elastomeric foam.

2. The apparatus of claim 1 wherein said first (46, 40, 50) and said second (48, 40, 50) elastomeric foam buttresses comprises an aliphatic polyester.

3. The apparatus of claim 2 wherein said aliphatic polyester is selected from the group consisting of copolymers of epsilon-caprolactone and glycolide, epsilon-caprolactone and lactide, para-dioxanone and lactide, epsilon-caprolactone and para-dioxanon, para-dioxanone and trimethylene carbonate, trimethylene carbonate and glycolide, and trimethylene carbonate and lactide.

4. The apparatus of claim 1 further comprising an adhesive (45) disposed upon said second (54) surface of said first (46, 40, 50) and second (48, 40, 50) foam buttresses to provide releasable attachment of said foam buttresses to said surgical stapling device (30) upon contact therewith.

5. The apparatus of claim 4 wherein said adhesive (45) is biocompatible.

6. The apparatus of claim 5 wherein said adhesive (45) is biodegradable.

7. The apparatus of claim 1 wherein said retaining means comprises a retention channel (21, 23) integral with and between said first (18) and second (20) guide channel walls and said receiving means (22) for cooperating with said foam buttresses to provide retention of said foam buttresses on said receiving (22) means prior to contact with said stapling device (30).

8. The apparatus of claim 7, wherein said first (46, 40) and second (48, 40) foam buttresses each comprise a rectangular body (42) with a series of tabs (44) extending laterally from the rectangular body (42) in an appropriate position to fit within and cooperate with the retention channel (21, 23).

9. The apparatus of claim 7 wherein said first (46, 40) and second (48, 40) foam buttresses further comprise means for cooperating with said retention channels (21, 23) to provide releasable retention of said buttresses on said receiving means (22) prior to contact with said stapling device (30).

10. The apparatus of claim 9 wherein said means for cooperating with said retention channels (21, 23) comprises tabs (44) integral with and extending laterally from said first (46, 40) and second (48, 40) foam buttresses in sufficient number and location along said foam buttresses to provide retention of said buttresses on said receiving means (22).

11. The apparatus of claim 10 wherein said tabs (44) are square, rectangular, half circular, trapezoid or triangular.

12. The apparatus of claim 1 wherein said retaining means comprises a biocompatible adhesive disposed between said receiving means and said first surface of said first and second foam buttresses.

13. The apparatus of claim 12 wherein said adhesive is biodegradable.

## Patentansprüche

1. Vorrichtung zum Ausstatten eines chirurgischen Klammergeräts (30), um verstärkte chirurgische Befestigungsnahtlinien zu liefern, umfassend:
ein Ausrichtemittel (10), das einen im wesentlichen ebenen Rahmen (16) umfasst, wobei der Rahmen umfasst:
eine erste Oberfläche (12) mit einer ersten Führungskanalwand (18), die sich davon erstreckt, und eine zweite Oberfläche (14), die entgegengesetzt zur ersten Oberfläche ist und eine zweite Führungskanalwand (20) umfasst, die sich davon erstreckt; und
ein Aufnahmemittel (22) mit ersten (24) und zweiten (26) entgegengesetzten Oberflächen,
wobei die erste Führungskanalwand (18) und die erste Oberfläche (24) des Aufnahmemittels (22) einen ersten Führungskanal definieren und die zweite Führungskanalwand (20) und die zweite Oberfläche (26) des Aufnahmemittels (22) einen zweiten Führungskanal definieren,
eine erste chirurgische Versteifung (46, 40, 50) mit einer ersten (52) Oberfläche zum Kontaktieren des Aufnahmemittels (22) und einer zweiten (54) Oberfläche, entgegengesetzt zur ersten (52) Oberfläche zum Kontaktieren des Klammergeräts (30), wobei die erste Versteifung (46, 40, 50) in dem ersten Führungskanal und auf der ersten Oberfläche (24) des Aufnahmemittels (22) angeordnet ist,
eine zweite chirurgische Versteifung (48, 50) mit einer ersten (52) Oberfläche zum Kontaktieren des Aufnahmemittels (22) und einer zweiten (54) Oberfläche entgegengesetzt zur ersten (52) Oberfläche zum Kontaktieren des Klammergeräts (30), wobei die zweite Versteifung (48, 40, 50) in dem zweiten Führungskanal und auf der zweiten Oberfläche (26) des Aufnahmemittels (22) angeordnet ist; und
ein Mittel zum Halten der ersten (46, 40, 50) und zweiten (48, 40, 50) Versteifungen auf den ersten (24) und zweiten (26) Oberflächen des Aufnahmemittels (22), **dadurch gekennzeichnet, dass** das Ausrichtemittel (10) als ein einheitlicher Körper ausgebildet ist und die ersten (46, 40, 50) und zweiten chirurgischen Versteifungen (48) aus einem elastomeren Schaum hergestellt sind.

2. Vorrichtung nach Anspruch 1, **dadurch gekennzeichnet, dass** die erste (46, 40, 50) und die zweite (48, 40, 50) elastomere Schaumversteifüng einen aliphatischen Polyester umfassen.

3. Vorrichtung nach Anspruch 2, **dadurch gekennzeichnet, dass** der aliphatische Polyester ausgewählt ist aus der Gruppe bestehend aus Copolymeren von epsilon-Caprolacton und Glycolid, epsilon-Caprolacton und Lactid, para-Dioxanon und Lactid, epsilon-Caprolacton und para-Dioxanon, Para-Dioxanon und Trimethylencarbonat, Trimethylencarbonat und Glycolid sowie Trimethylencarbonat und Lactid.

4. Vorrichtung nach Anspruch 1, ferner umfassend einen Klebstoff (45), der auf der zweiten (54) Oberfläche der ersten (46, 40, 50) und zweiten (48, 40, 50) Schaumversteifungen angeordnet ist, um eine lösbare Befestigung besagter Schaumversteifungen an dem chirurgischen Klammergerät (30) bei Kontakt damit zu liefern.

5. Vorrichtung nach Anspruch 4, **dadurch gekennzeichnet, dass** der Klebstoff (45) biologisch verträglich ist.

6. Vorrichtung nach Anspruch 5, **dadurch gekennzeichnet, dass** der Klebstoff (45) biologisch abbaubar ist.

7. Vorrichtung nach Anspruch 1, **dadurch gekennzeichnet, dass** das Haltemittel einen Sicherungskanal (21, 23) integral mit und zwischen den ersten (18) und zweiten (20) Führungskanalwänden und dem Aufnahmemittel (20) zum Zusammenwirken mit den Schaumversteifungen aufweist, um für eine Sicherung der Schaumversteifungen an dem Aufnahme (22)-Mittel vor Kontakt mit dem Klammergerät (12) zu sorgen.

8. Vorrichtung nach Anspruch 7, **dadurch gekennzeichnet, dass** die ersten (46, 40) und zweiten (48, 40) Schaumversteifungen jeweils einen rechteckigen Körper (42) mit einer Reihe von Zungen umfassen, die sich seitlich von dem rechteckigen Körper (42) in einer geeigneten Position erstrecken, um in den Sicherungskanal (21, 23) zu passen und damit zusammenzuwirken.

9. Vorrichtung nach Anspruch 7, **dadurch gekennzeichnet, dass** die ersten (46, 40) und zweiten (48, 40) Schaumversteifungen ferner ein Mittel zum Zusammenwirken mit dem Sicherungskanal (21, 23) umfassen, um für eine lösbare Sicherung der Versteifungen an dem Aufnahmemittel (22) vor Kontakt mit dem Klammergerät (30) zu sorgen.

10. Vorrichtung nach Anspruch 9, **dadurch gekennzeichnet, dass** das Mittel zum Zusammenwirken mit den Sicherungskanälen (21, 23) Zungen (44), die mit den ersten (46, 40) und zweiten (48, 40) Schaumversteifungen integral ausgebildet sind und sich davon seitlich erstrecken, in ausreichender Anzahl und an Stellen entlang der Schaumversteifungen umfasst, um für Sicherung der Versteifungen an dem Aufnahmemittel (22) zu sorgen.

11. Vorrichtung nach Anspruch 10, **dadurch gekennzeichnet, dass** die Zungen (44) quadratisch, rechteckig, halbkreisförmig, trapezförmig oder dreieckig sind.

12. Vorrichtung nach Anspruch 1, **dadurch gekennzeichnet, dass** das Haltemittel einen biologisch verträglichen Klebstoff umfasst, der zwischen dem Aufnahmemittel und der ersten Oberfläche der ersten und zweiten Schaumversteifungen angeordnet ist.

13. Vorrichtung nach Anspruch 12, **dadurch gekennzeichnet, dass** der Klebstoff biologisch abbaubar ist.

## Revendications

1. Appareil pour équiper un dispositif d'agrafage chirurgical (30) afin de fournir des lignes de suture de fixations chirurgicales renforcées, comprenant :
→ des moyens d'alignement (10) qui comprennent un châssis (16) sensiblement plan, ledit châssis comprenant :
→ une première surface (12) comprenant une première paroi de canal de guidage (18) s'étendant à partir de celle-ci et une seconde surface (14) apposée à ladite première surface et comprenant une seconde paroi de canal de guidage (20) s'étendant à partir de celle-ci ; et
→ des moyens de réception (22) comprenant des première (24) et seconde (26) surfaces apposées,
→ ladite première paroi de canal de guidage (18) et ladite première surface (24) desdits moyens de réception (22) définissant un premier canal de guidage et ladite seconde paroi de canal de guidage (20) et ladite seconde surface (26) desdits moyens de réception (22) définissant un second canal de guidage,
→ un premier renfort chirurgical (46, 40, 50) comprenant une première surface (52) pour mettre en contact lesdits moyens de réception (22) et une seconde surface (54) apposée à ladite première surface (52) pour mettre en contact ledit dispositif d'agrafage (30), ledit premier renfort (46, 40, 50) étant disposé à l'intérieur dudit premier canal de guidage et sur ladite première surface (24) desdits moyens de réception (22),
→ un second renfort chirurgical (48, 50) comprenant une première surface (52) pour mettre en contact lesdits moyens de réception (22) et une seconde surface (54) apposée à ladite première surface (52) pour mettre en contact ledit dispositif d'agrafage (30), ledit second renfort (48, 40, 50) étant disposé à l'intérieur dudit second canal de guidage et sur ladite seconde surface (26) desdits moyens de réception (22) ; et
→ des moyens pour retenir lesdits premier (46, 40, 50) et second (48, 40, 50) renforts sur lesdites première (24) et seconde (26) surfaces desdits moyens de réception (22), **caractérisé en ce que** les moyens d'alignement (10) sont formés comme un corps unitaire et les premier (46, 40, 50) et second renforts chirurgicaux (48) sont réalisés avec une mousse élastomère.

2. Appareil selon la revendication 1, dans lequel lesdits premiers (46, 40, 50) et lesdits seconds (48, 40, 50) renforts en mousse élastomère comprennent un polyester aliphatique.

3. Appareil selon la revendication 2, dans lequel ledit polyester aliphatique est choisi dans le groupe comprenant les copolymères d'epsilon caprolactone et de glycolide, d'epsilon caprolactone et de lactide, de para-dioxanone et de lactide, d'epsilon caprolactone et de para-dioxanone, de para-dioxanone et de carbonate de triméthylène, de carbonate de triméthylène et de glycolide et de carbonate de triméthylène et de lactide.

4. Appareil selon la revendication 1, comprenant en outre un adhésif (45) disposé sur ladite seconde surface (54) desdits premier (46, 40, 50) et second (48, 40, 50) renforts en mousse pour fournir la fixation amovible desdits renforts en mousse sur ledit dispositif d'agrafage chirurgical (30) suite au contact avec celui-ci.

5. Appareil selon la revendication 4, dans lequel ledit adhésif (45) est biocompatible.

6. Appareil selon la revendication 5, dans lequel ledit adhésif (45) est biodégradable.

7. Appareil selon la revendication 1, dans lequel lesdits moyens de retenue comprennent un canal de retenue (21, 23) solidaire de et entre lesdites première (18) et seconde (20) parois de canal de guidage et lesdits moyens de réception (22) pour coopérer avec lesdits renforts en mousse pour fournir la retenue desdits renforts en mousse sur lesdits moyens de réception (22) avant d'entrer en contact avec ledit dispositif d'agrafage (30).

8. Appareil selon la revendication 7, dans lequel lesdits premier (46, 40) et second (48, 40) renforts en mousse comprennent chacun un corps rectangulaire (42) avec une série de languettes (44) s'étendant latéralement à partir du corps rectangulaire (42) dans une position appropriée pour s'adapter à l'intérieur et coopérer avec le canal de retenue (21, 23).

9. Appareil selon la revendication 7, dans lequel lesdits premier (46, 40) et second (48, 40) renforts en mousse comprennent en outre des moyens pour coopérer avec lesdits canaux de retenue (21, 23) pour fournir la retenue amovible desdits renforts sur lesdits moyens de réception (22) avant d'entrer en contact avec ledit dispositif d'agrafage (30).

10. Appareil selon la revendication 9, dans lequel lesdits moyens pour coopérer avec lesdits canaux de retenue (21, 23) comprennent des languettes (44) solidaires de et s'étendant latéralement à partir desdits premier (46, 40) et second (48, 40) renforts en mousse en nombre et emplacement suffisants le long desdits renforts en mousse pour fournir la retenue desdits renforts sur lesdits moyens de retenue (22).

11. Appareil selon la revendication 10, dans lequel lesdites languettes (44) sont carrées, rectangulaires, semi-circulaires, trapézoïdales ou triangulaires.

12. Appareil selon la revendication 1, dans lequel lesdits moyens de retenue comprennent un adhésif biocompatible disposé entre lesdits moyens de réception et ladite première surface desdits premier et second renforts en mousse.

13. Appareil selon la revendication 12, dans lequel ledit adhésif est biodégradable.
